# EUROPEAN PATENT APPLICATION

(11) **EP 4 722 330 A1**
(43) Date of publication of application: **08.04.2026**
(21) Application number: 24814568.2
(22) Date of filing: 31.05.2024
(51) Int. Cl.: C12M 1/00

(54) **EXTRACTION APPARATUS FOR SAMPLE**

(30) Priority: 31.05.2023 CN 202310637950
(71) Applicant: Nanjing GenScript Biotech Co., Ltd., Nanjing, Jiangsu 211100 (CN)
(72) Inventor: WEI, Jinwen, Nanjing, Jiangsu 211100 (CN); SUN, Zhenbo, Nanjing, Jiangsu 211100 (CN); ZHENG, Cheng, Nanjing, Jiangsu 211100 (CN); FANG, Jiujin, Nanjing, Jiangsu 211100 (CN); ZHOU, Yang, Nanjing, Jiangsu 211100 (CN); ZHANG, Zhun, Nanjing, Jiangsu 211100 (CN); QIAO, Yuehua, Nanjing, Jiangsu 211100 (CN); KE, Lanlan, Nanjing, Jiangsu 211100 (CN)
(74) Representative: Latscha Schöllhorn Partner AG
(86) International application number: PCT/CN2024/096527
(87) International publication number: WO 2024/245373

(57) **Abstract**

An extraction apparatus (10) for a sample, the extraction apparatus including: a sample processing device (100), the sample processing device (100) including a magnetic attraction and mixing mechanism (120) for performing mixing and magnetic attraction on a sample; a mechanical arm device (400) including a set of guide rails (410) and an operating arm (420) movable along the set of guide rails (410), the set of guide rails (410) including a first guide rail (411) and a second guide rail (412) perpendicular to each other in a horizontal plane, and the operating arm (420) being movable in a direction perpendicular to the horizontal plane; and a consumable assembly (200), where the operating arm (420) is movable between the sample processing device (100) and the consumable assembly (200) along the set of guide rails (410).

## Description

### Cross-Reference to Related Applications

The present invention claims priority to Chinese Patent Application No. 202310637950.4 filed on May 31, 2023, which is incorporated herein by reference in its entirety.

### Technical Field

The present description relates to the technical field of biological devices, more particularly, to an extraction apparatus for a sample.

### Background Art

Extraction or purification of target biological substances (e.g., plasmids) is a crucial step in molecular biology and medical research, and the performance of extraction or purification directly affect the progress and results of research and diagnosis. In modern bioengineering, plasmids are used as vectors for genetic engineering. DNA fragments are inserted into plasmids by experimenters, and then replicated in large quantities by means of bacteria, and finally the plasmids are extracted from the bacteria. The current extraction or purification process of the target biological substances (e.g., plasmids) is usually carried out by a combination of manual and mechanical means, but the manual operation part accounts for a high proportion of the entire process, which is labour-intensive and time-consuming. Moreover, since manual operations often involve individual variations, which will affect the accuracy of the results and exhibit suboptimal consistency in process execution, the throughput of detection is constrained, making it difficult to meet the demand for extracting or purifying high-throughput samples amid the current rapid development of bioengineering.

Therefore, it is necessary to provide an extraction apparatus for a biological substance, so as to effectively improve the effect and efficiency of the extraction or purification of target biological substances.

### Summary

One of the embodiments of the present description provides an extraction apparatus for a sample, the extraction apparatus including: a sample processing device, including a magnetic attraction and mixing mechanism for performing mixing and magnetic attraction on a sample; a mechanical arm device, including a set of guide rails and an operating arm movable along the set of guide rails, the set of guide rails including a first guide rail and a second guide rail perpendicular to each other in a horizontal plane, and the operating arm being movable in a direction perpendicular to the horizontal plane; and a consumable assembly, wherein the operating arm is movable between the sample processing device and the consumable assembly along the set of guide rails.

One of the embodiments of the present description provides a method of use of an extraction apparatus, the method of use including: controlling a mechanical arm device to add a sample into a first processing chamber of a processing box and to add a first reagent into the first processing chamber; controlling a mixing assembly to perform a first mixing on the processing box; controlling the mechanical arm device to add first magnetic beads into the first processing chamber; controlling a magnetic assembly to perform a first magnetic attraction on the first magnetic beads in the first processing chamber; controlling the mechanical arm device to transfer a first supernatant from the first processing chamber to a second processing chamber of the processing box and to add second magnetic beads into the second processing chamber; controlling the mixing assembly to perform a second mixing on the processing box; controlling the magnetic assembly to perform a second magnetic attraction on the second magnetic beads in the second processing chamber; controlling the mechanical arm device to remove a second supernatant from the second processing chamber while retaining the attracted second magnetic beads; and performing elution on the second magnetic beads and controlling the mechanical arm device to collect a third supernatant from the second processing chamber.

### Brief Description of the Drawings

The present description will be further illustrated by way of exemplary embodiments, and these exemplary embodiments will be described in detail with reference to the accompanying drawings. These embodiments are not limiting, and in these embodiments, the same reference numerals represent the same structures, in which:
Fig. 1 is a schematic structural view of an extraction apparatus according to some embodiments of the present description;
Fig. 2 is a schematic structural view of a processing box according to some embodiments of the present description;
Fig. 3 is a schematic structural view of a sample processing device according to some embodiments of the present description;
Fig. 4 is a schematic structural view of the processing box connected to a mixing assembly according to some embodiments of the present description;
Fig. 5 is a schematic cross-sectional view of the processing box connected to the mixing assembly according to some embodiments of the present description;
Fig. 6 is a schematic cross-sectional view of the processing box connected to the mixing assembly according to some other embodiments of the present description;
Fig. 7 is a schematic structural view of the processing box connected to a magnetic assembly according to some embodiments of the present description;
Fig. 8 is a schematic cross-sectional view of the processing box connected to the magnetic assembly according to some embodiments of the present description;
Fig. 9 is a schematic structural view of a consumable assembly and a magnetic attraction and mixing mechanism according to some embodiments of the present description;
Fig. 10 is a schematic structural view of a mixing mechanism according to some embodiments of the present description;
Fig. 11 is a schematic cross-sectional view of the mixing mechanism according to some embodiments of the present description;
Fig. 12 is a schematic structural view of a mechanical arm device according to some embodiments of the present description;
Fig. 13 is a schematic structural view of a set of guide rails according to some embodiments of the present description;
Fig. 14 is a schematic structural view of a first guide rail connected to a second guide rail according to some embodiments of the present description;
Fig. 15 is a schematic structural view of the second guide rail according to some embodiments of the present description;
Fig. 16 is a partial schematic view of the second guide rail according to some embodiments of the present description;
Fig. 17 is a schematic structural view of a lifting guide rail connected to an operating arm according to some embodiments of the present description;
Fig. 18 is a schematic structural view of the lifting guide rail according to some embodiments of the present description; and
Fig. 19 is an exemplary flow chart of a method of use according to some embodiments of the present description.

Reference signs: extraction apparatus 10; sample processing device 100; processing box 110; processing chamber 111; first processing chamber 112; second processing chamber 113; mounting hole 114; isolation member 115; magnetic attraction and mixing mechanism 120; magnetic assembly 130; magnet 131; first magnet 1311; second magnet 1312; magnet driving mechanism 132; first driving motor 133; lead screw 134; linkage 135; lead screw nut 136; nut track 137; magnet support member 1391; linear guide rail 1392; base 1393; mixing assembly 140; second driving motor 141; mixing platform 142; eccentric shaft 1421; first mixing bearing 1431; second mixing bearing 1432; third mixing bearing 1433; first direction adapter plate 1441; second direction adapter plate 1442; first mixing guide rail 1451; second mixing guide rail 1452; motor fixing plate 146; support plate 147; connecting shaft 148; coupling 149; consumable assembly 200; reagent kit 210; cavity 211; pipette tip box 220; pipette tip 221; consumable support 230; target biological substance storage box 240; chamber 241; sample preprocessing box 250; magnetic bead storage box 260; holding chambers 261; waste liquid box 270; mixing mechanism 300; third driving motor 310; second mixing platform 320; second eccentric shaft 321; fourth mixing bearing 331; fifth mixing bearing 332; sixth mixing bearing 333; third direction adapter plate 341; fourth direction adapter plate 342; third mixing guide rail 351; fourth mixing guide rail 352; second motor fixing plate 360; second support plate 370; second base 380; mechanical arm device 400; set of guide rails 410; first guide rail 411; second guide rail 412; operating arm 420; pump connecting plate 421; liquid pump 422; support frame 430; first synchronizing belt 441; first guide rail motor 442; drive shaft 443; first pulley 444; second synchronizing belt 451; second guide rail motor 452; crossbeam 453; second guide rail support plate 454; second guide rail slider 455; second motor support plate 456; idler 457; second pulley 458; lifting motor 461; lifting lead screw 462; lifting guide rail 463; lifting guide rail support plate 464; top support plate 4641; bottom support plate 4642; lifting guide rail slider 465; third synchronizing belt 466; lifting nut 467; second optocoupler 511; second optocoupler barrier 512; third optocoupler 521; third optocoupler barrier 522; fourth optocoupler 531; fourth optocoupler barrier 532; fifth optocoupler 541; fifth optocoupler barrier 542; sixth optocoupler 551; sixth optocoupler barrier 552; scanning sensor 560.

### Detailed Description of Embodiments

For a clear description of the technical solutions of the embodiments in the present description, the accompanying drawings required for describing the embodiments will be briefly described below. Apparently, the accompanying drawings in the following description show merely some examples or embodiments of the present description, and those of ordinary skill in the art may still apply the present description to other similar scenarios according to these accompanying drawings without any creative effort. Unless obvious from the linguistic context or otherwise stated, the same reference signs in the drawings represent the same structure or operation.

As shown in the present description and the claims, the words "one", "a", "an" and/or "the" do not specifically refer to the singular, but may also include the plural, unless the context clearly indicates otherwise. Generally, the terms "comprising" and "including" only elicit explicitly identified steps and elements, which, however, are not exclusive in the list. In other words, said method or device may further include other steps or elements.

The present description provides an extraction apparatus for a biological substance, which can be used to extract and/or purify a target biological substance in a sample, so that it is possible to significantly reduce the workload of experimenters and shorten the work time. Moreover, the extraction apparatus is mechanically automated throughout its work, with high consistency in repeated operations and better overall extraction and/or purification results. In some embodiments, the extraction apparatus can be used for high-throughput sample processing, i.e., performing extraction and/or purification on a plurality of samples simultaneously, to significantly improve the efficiency of extraction and/or purification. The target biological substance in the present description is a substance in the sample that is to be extracted (or separated) and/or purified. In some embodiments, the target biological substance includes a plasmid (e.g., a DNA plasmid, or an RNA plasmid), a protein, a nucleic acid, and any other feasible biological substances. For example, the extraction apparatus can be used to process a sample to extract and/or purify a plasmid. For another example, the extraction apparatus can be used to process a sample to extract and/or purify a protein. For still another example, the extraction apparatus is used to process a sample to extract and/or purify a nucleic acid. In some embodiments, the sample may be a microorganism (including bacteria, viruses, fungi, etc. such as escherichia coli) culture solution, blood, saliva, an animal tissue, a plant, food, or any other feasible samples. Extraction is the separation of the target biological substance from other impurities in a sample (e.g., a culture solution). Purification is the further purification of the separated target biological substance. It should be understood that application scenarios of the extraction apparatus in the description of the present application are merely some examples or embodiments of the present application. Those of ordinary skill in the art may still apply the present application to other similar scenarios according to the accompanying drawings without any creative effort.

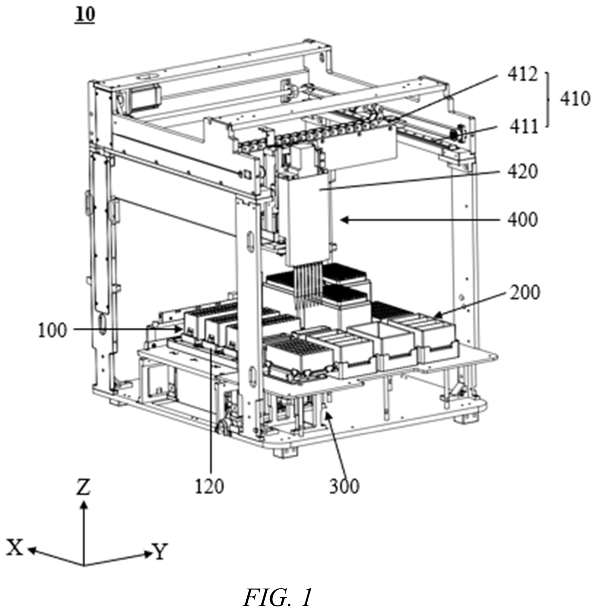

Fig. 1 shows a schematic structural diagram exemplarily showing an extraction apparatus 10 for a sample. For ease of description, the various assemblies and/or devices of the extraction apparatus 10 are further described using a coordinate system as shown in Fig. 1. The coordinate system (e.g., a Cartesian coordinate system) includes an X-axis, a Y-axis, and a Z-axis. The Z-axis is parallel to a vertical direction (for example, being perpendicular to the ground), the X-axis and the Y-axis are parallel to a horizontal direction (for example, being parallel to the ground), and the X-axis is perpendicular to the Y-axis. The Y-axis direction is also referred to as a first direction. The X-axis direction is also referred to as a second direction. The Z-axis direction is also referred to as a third direction.

In some embodiments, as shown in Fig. 1, the extraction apparatus 10 may include a sample processing device 100, a mechanical arm device 400, and a consumable assembly 200. The sample processing device 100 includes a magnetic attraction and mixing mechanism 120. The magnetic attraction and mixing mechanism 120 may be configured to perform mixing and magnetic attraction on a sample. The mechanical arm device 400 may include a set of guide rails 410 and an operating arm 420 movable along the set of guide rails 410. The set of guide rails 410 may include a first guide rail 411 and a second guide rail 412 which are located on a horizontal plane and are perpendicular to each other. The operating arm 420 may extend and retract in a direction perpendicular to the horizontal plane so that the operating arm 420 can move between the sample processing device 100 and the consumable assembly 200 along the set of guide rails 410.

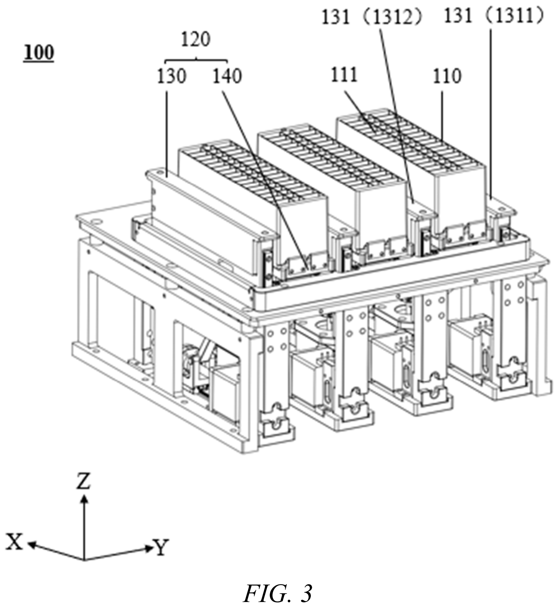

In the present description, mixing is to mix the sample by shaking or vibration. Magnetic attraction is the use of magnetic attraction action to separate impurities or a target biological substance from the sample. By way of example only, during the process of extracting and/or purifying a sample, it is necessary to add specific reagents into the sample to achieve different functions (e.g., lysis reaction with the sample, or separation of magnetic beads from a magnetic bead conjugate) and add magnetic beads to the sample, such that the magnetic beads are conjugated to impurities or a target biological substance in the sample, in order to separate the target biological substance or impurities from the solution. The magnetic bead in the present description is a magnetic particle that has a surface-active group. Under certain conditions, the surface-active group of the magnetic bead can be conjugated to (e.g., be coupled with) the target biological substance (e.g., a substance to be extracted) or impurities (e.g., substances other than the substance to be extracted) in the sample. Under the action of an external magnetic field (e.g., a magnetic field formed by a magnet 131 in Fig. 3), the magnetic beads can be subjected to magnetic attraction (i.e., magnetically attracted), thereby achieving separation of the active substance from the impurities and/or purification of the active substance.

In some embodiments, the corresponding type of magnetic beads may be selected depending on the particle size, the dispersity, the magnetic response time, and the coating matrices of the magnetic beads. The type of the magnetic beads is not limited here. In some embodiments, the types of reagents may include solvents for reacting with samples and for washing magnetic bead conjugates, reagents for eluting magnetic bead conjugates to separate magnetic beads, etc. The types of reagents are not limited here. Taking plasmid extraction by means of bacterial sludge as an example, the reagent may include a bacterial sludge lysis solution, a washing solution, an eluent, etc. The magnetic beads may include magnetic beads capable of being conjugated to impurities obtained after bacterial sludge is lysed, magnetic beads capable of being conjugated to a plasmid, etc. In some embodiments, the magnetic attraction and mixing mechanism 120 mixes the magnetic beads with the sample to improve the efficiency of conjugation of the magnetic beads to the impurities and/or the target biological substance in the sample.

In some embodiments, the types of reagents may include solvents for reacting with samples and for washing magnetic bead conjugates, reagents for eluting magnetic bead conjugates to separate magnetic beads, etc. The types of reagents are not limited here. Taking plasmid extraction by means of bacterial sludge as an example, the reagent may include a bacterial sludge lysis solution, a washing solution, an eluent, etc. The magnetic beads may include magnetic beads capable of being conjugated to impurities obtained after bacterial sludge is lysed, magnetic beads capable of being conjugated to a plasmid, etc. In some embodiments, when a reagent is added for reaction with the sample, the magnetic attraction and mixing mechanism 120 may be used to mix the reagent with the sample to improve the efficiency of reaction of the sample. When a reagent is added for washing a magnetic bead conjugate, the magnetic attraction and mixing mechanism 120 may be configured to mix the reagent with the sample to improve the washing efficiency. When a reagent is added for eluting the magnetic bead conjugate to separate the magnetic beads, the magnetic attraction and mixing mechanism 120 may be configured to mix the reagent with the sample to improve the efficiency of separation of the magnetic beads from the magnetic bead conjugate.

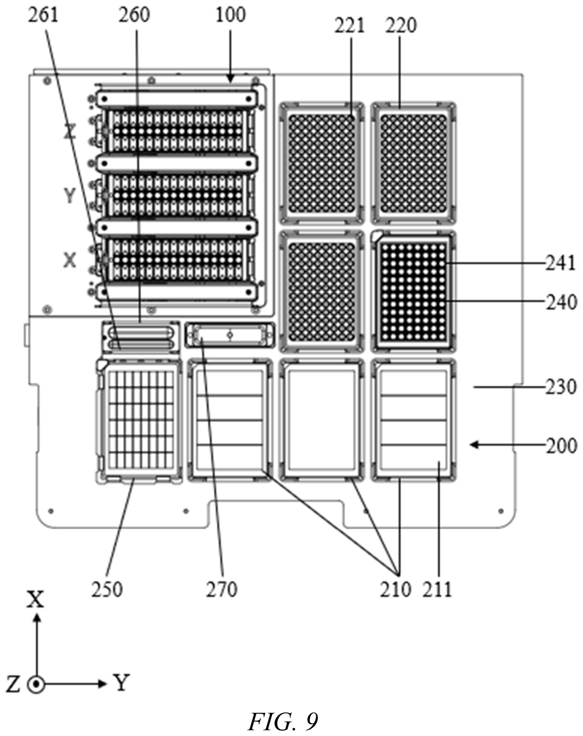

The consumable assembly 200 may include a consumable and a carrier thereof for use during the processing of the sample. The operating arm 420 may be moved between the consumable assembly 200 and the sample processing device 100 by the cooperation with the set of guide rails 410 to perform operations including liquid transfer, waste liquid collection, pipette tip mating, and magnetic bead addition. More details about the consumable assembly 200 can be found in the description of Fig. 9 and its embodiments.

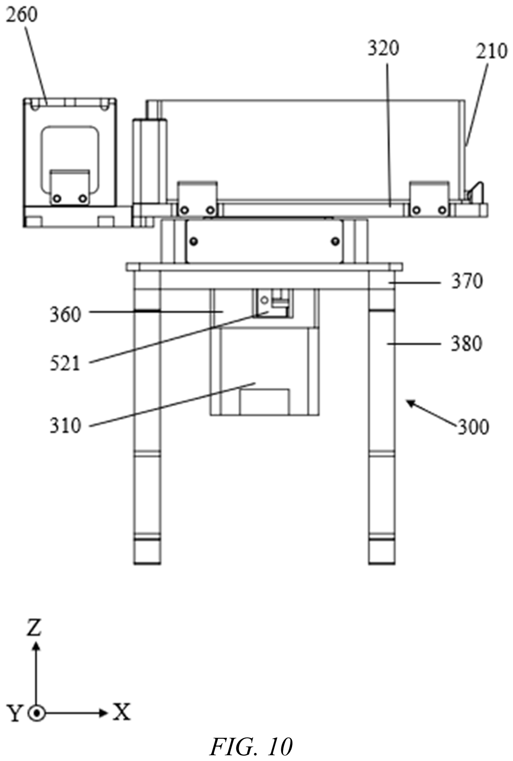

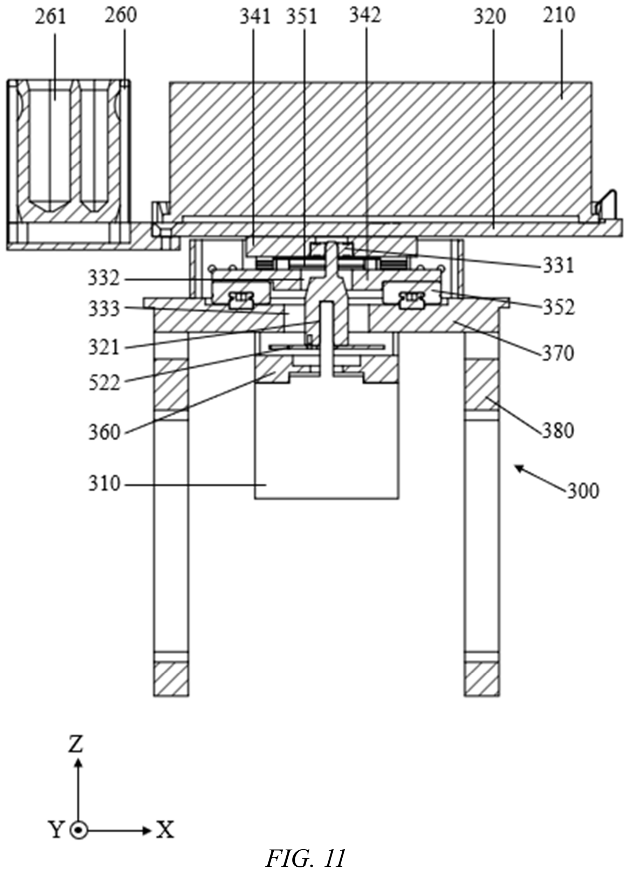

In some embodiments, the extraction apparatus 10 may further include a mixing mechanism 300. The mixing mechanism 300 may be configured to preprocess the sample prior to extraction and/or purification of the sample to make the sample conform to experimental requirements. More details about the mixing mechanism 300 can be found in the description of Figs. 10-11 and their embodiments.

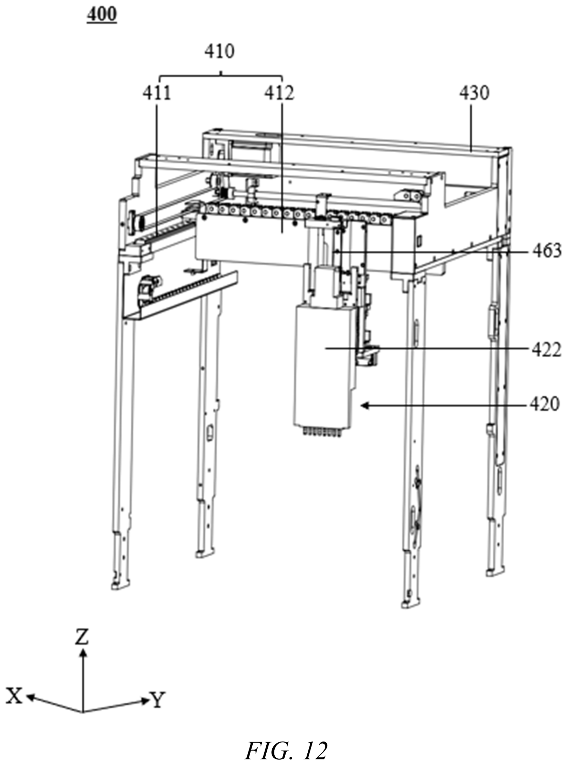

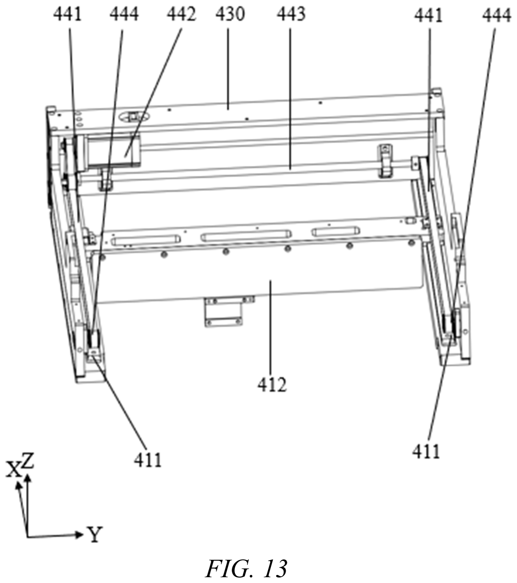

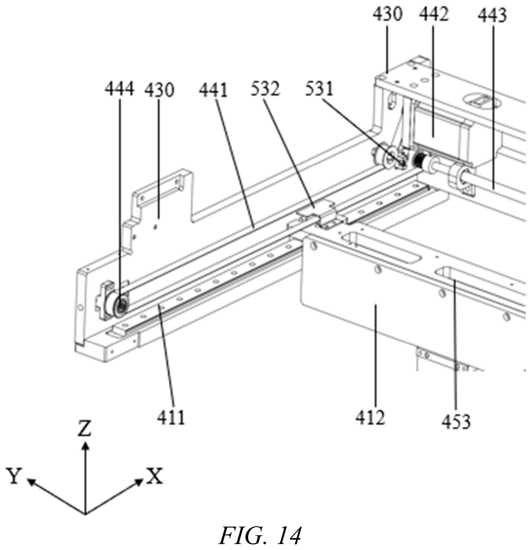

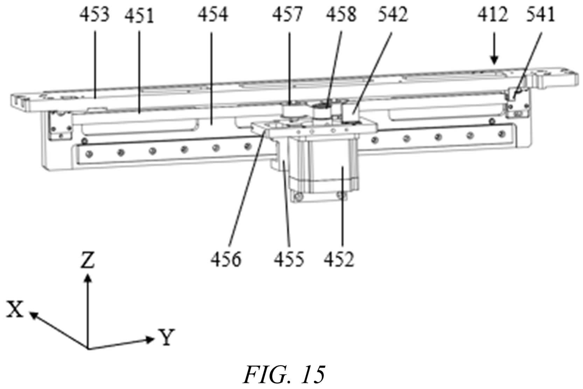

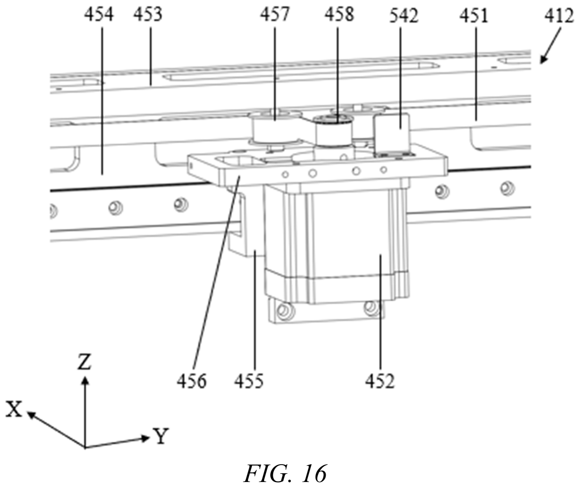

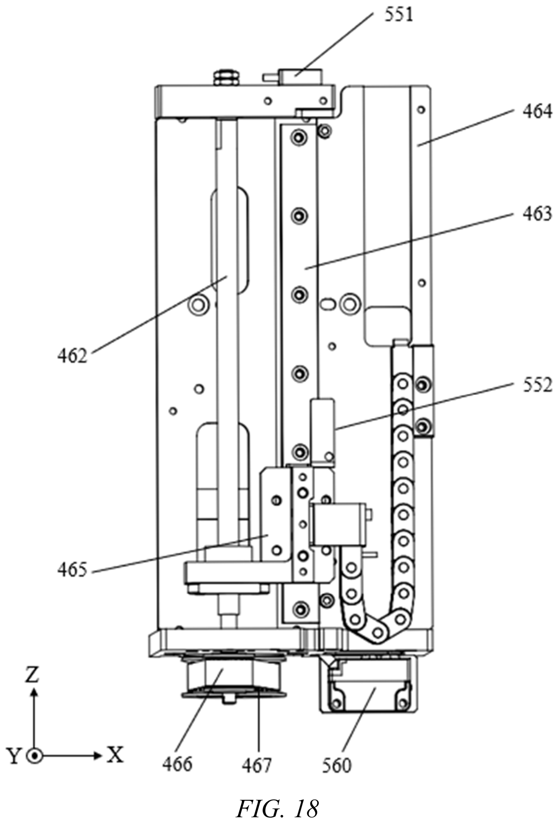

The operating arm 420 may be configured to add, transfer, and collect a specific substance (e.g., a supernatant, or a target biological substance) during the extraction and/or purification of the sample. The set of guide rails 410 may be configured to allow the operating arm 420 to move and limit a movement path of the operating arm 420. More details about the mechanical arm device 400 can be found in the description of Figs. 12-18 and their embodiments.

In some cases, the sample processing device 100 of the extraction apparatus 10 provided in the present description can attract the magnetic beads in a sample that are conjugated to the impurities or the target biological substance, thereby facilitating the separation of the impurities or the target biological substance from the solution by means of the mechanical arm device 400. In addition, the extraction apparatus 10 can mix the reagent and/or the magnetic beads added into the sample to improve the efficiency and effectiveness of the extraction and/or purification of the target biological substance. In some other cases, since the operating arm 420 of the present description can not only move in the horizontal plane along the first guide rail 411 and the second guide rail 412, but also extend and retract in the direction perpendicular to the horizontal plane, the operating arm 420 can move between the sample processing device 100 and the consumable assembly 200 along the set of guide rails 410. This not only makes the layout of the consumable assembly 200 more compact, saves the overall space size of the extraction apparatus 10, but also enables the operating arm 420 to perform such operations as liquid transfer, waste liquid collection, pipette tip mating, and magnetic bead addition more efficiently and conveniently.

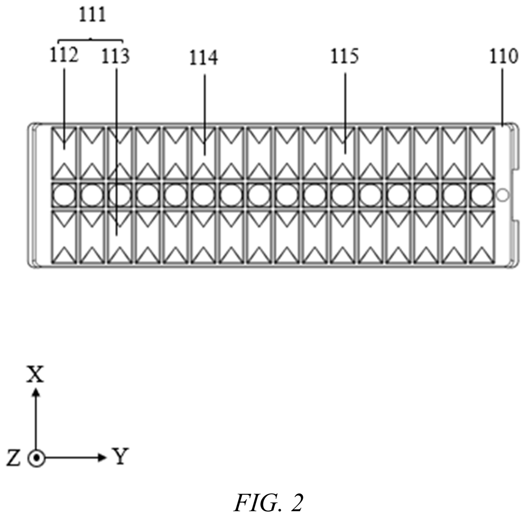

In some embodiments, as shown in Figs. 1-3, the sample processing device 100 may further include a processing box 110. The processing box 110 may include two sets of processing chambers 111. The processing chambers 111 may be configured to process the sample. In some embodiments, one of the two sets of processing chambers 111 is used to process the sample at the same moment.

In some cases, during the extraction and/or purification of the target biological substance using the extraction apparatus 10 provided in the present description, when two processing chambers 111 are used to process the sample simultaneously, due to the need to perform magnetic attraction on the impurities or the target biological substance in the processing chambers 111 using the magnetic attraction and mixing mechanism 120, performing magnetic attraction on the sample in one of the processing chambers 111 may affect the sample in the other processing chamber 111. By way of example only, as shown in Fig. 3, the magnetic beads in one of the processing chambers 111 (which may be referred to as a first processing chamber, e.g., a first processing chamber 112) are conjugated to the impurities, and the magnetic beads in the other processing chamber 111 (which may be referred to as a second processing chamber, e.g., a second processing chamber 113) are conjugated to the target biological substance. When the magnetic beads in the first processing chamber 112 are subjected to magnetic attraction, the magnetic beads in the second processing chamber 113 may be attracted, thereby affecting the sample in the second processing chamber 113. Therefore, in order to prevent the sample in the other set of processing chambers 111 from being affected, only one of the two sets of processing chambers 111 is used to process the sample at the same moment.

In some embodiments, each set of processing chambers 111 may include a plurality of mounting holes 114. The plurality of mounting holes 114 may be disposed along an extending direction of the processing chambers 111. The mounting hole 114 may be configured to contain the sample. The two sets of processing chambers 111 have the same number of mounting holes 114 which are in one-to-one correspondence. One-to-one correspondence here may mean that in the distribution direction of the two sets of processing chambers 111, any one of the mounting holes 114 in one set of processing chambers 111 is aligned with one of the mounting holes 114 in the other set of processing chambers 111, to facilitate the transfer of the sample from one set of processing chambers 111 to the other set of processing chambers 111 by a pipetting device (not shown). For example, as shown in Fig. 3, the first processing chamber 112 and the second processing chamber 113 each include 16 mounting holes 114. The 16 mounting holes 114 are disposed in the Y-axis direction. In the X-axis direction, one mounting hole 114 of the first processing chamber 112 is aligned with one mounting hole 114 of the second processing chamber 113.

In some embodiments, the two sets of processing chambers 111 may be spaced apart from each other. In some embodiments, the distance between the two sets of processing chambers 111 may be increased to reduce or avoid the influence on the sample in the other set of processing chambers 11 when performing magnetic attraction, so that the two sets of processing chambers 11 may be used to process the sample simultaneously, to improve the sample processing efficiency. For example, a gap of a preset distance may be provided between the two sets of processing chambers 111. In some embodiments, an isolation member 115 may be provided between the two sets of processing chambers 111 to reduce or block the magnetic force between the two sets of processing chambers 111 so that the two sets of processing chambers 111 may be used to process the sample simultaneously. In some embodiments, the isolation member 115 may include a magnetic shielding plate made of pure iron, an iron-nickel alloy, etc. In some embodiments, the isolation member 115 may not be a magnetic shielding member but physically separate the two sets of processing chambers 111. In some other embodiments, the two sets of processing chambers 111 may be disposed adjacent to each other.

It should be noted that the description of the number of processing boxes 110, the number of processing chambers 111 contained by the processing box 110, and the number of mounting holes 114 is for illustrative purposes only and is not intended to limit the number of processing boxes 110, the number of processing chambers 111 contained by the processing box 110 and the number of mounting holes 114 in the present description. For example, the processing box 110 may include only one processing chamber 111, or the processing box 110 may include three processing chambers 111, which are disposed in parallel at intervals. For another example, the number of processing boxes 110 may be one or plural, which can be adjusted according to actual requirements.

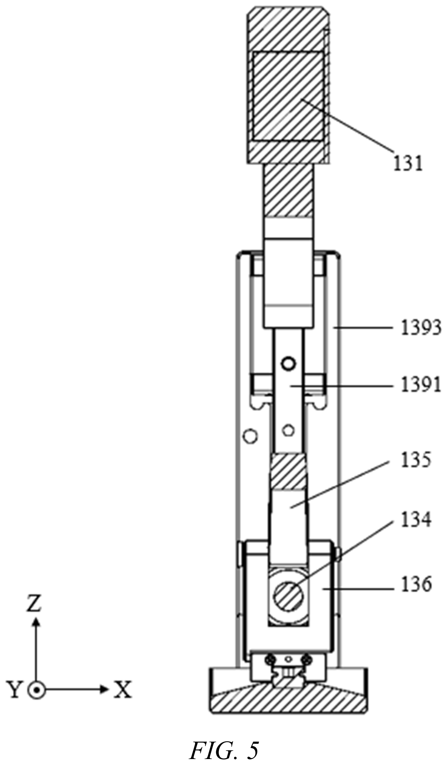

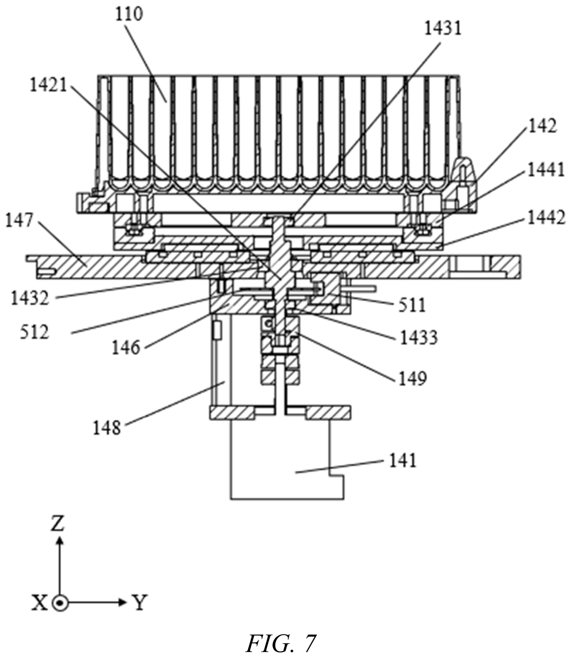

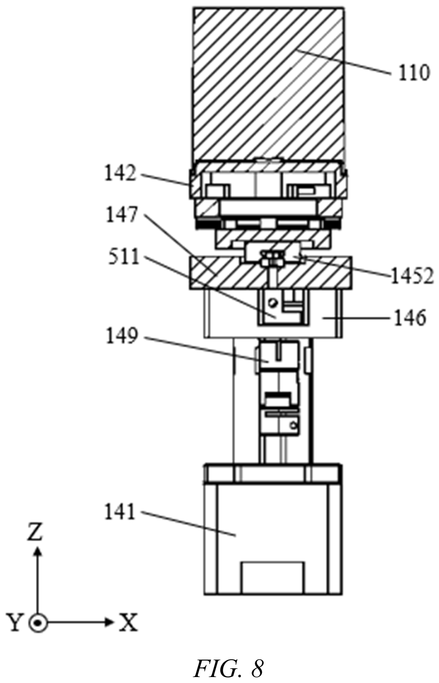

In some embodiments, as shown in Figs. 3-8, the magnetic attraction and mixing mechanism 120 may include a mixing assembly 140 and a magnetic assembly 130. The mixing assembly 140 may be configured to mix the sample, and the magnetic assembly 130 may be configured to magnetically attract the sample. The magnetic assembly 130 may include a magnet 131. The magnet 131 may be disposed on at least one side of the mixing assembly 140, and the processing box 110 may be disposed on the mixing assembly 140. In this embodiment, the magnet 131 may be configured to form an external magnetic field. After the magnetic beads are conjugated to the impurities or the target biological substance in the sample, the magnetic beads in the processing box 110 may be attracted under the action of the external magnetic field, such that the magnetic beads tightly fit against a side wall of the processing chamber 111 to facilitate the transfer of the remaining solution by means of the mechanical arm device 400, thereby separating the impurities or the target biological substance from the solution. In some embodiments, the magnet 131 has a predetermined magnetic attraction area. In some embodiments, the magnetic attraction area of the magnet 131 may be the area of a magnetic attraction surface facing the processing box 110 for magnetic attraction.

In some embodiments, as shown in Figs. 3-5, the magnet 131 may include a first magnet 1311 and a second magnet 1312, the processing box 110 may be disposed between the first magnet 1311 and the second magnet 1312, and the two sets of processing chambers 111 are arranged in the same direction as the first magnet 1311 and the second magnet 1312. By way of example only, the extending directions of the first processing chambers 112 and the second processing chambers 113 may both be parallel to the Y-axis direction, and the first processing chamber 112 and the second processing chamber 113 are arranged spaced apart in the X-axis direction. The first magnet 1311 and the second magnet 1312 are arranged spaced apart in the X-axis direction, the first magnet 1311 is disposed on a side of the first processing chambers 112 away from the second processing chambers 113, and the second magnet 1312 is disposed on a side of the second processing chambers 113 away from the first processing chambers112. In this embodiment, the first magnet 1311 may be configured to form a first external magnetic field for performing magnetic attraction on the impurities or the target biological substance in the first processing chambers 112. For example, after the magnetic beads in the first processing chambers 112 are conjugated to the impurities or the target biological substance in the sample, the magnetic beads may be attracted by the first magnet 1311 to separate the impurities or the target biological substance from the solution. The second magnet 1312 may be configured to form a second external magnetic field for performing magnetic attraction on the impurities or the target biological substance in the second processing chambers 113. For example, after the magnetic beads in the second processing chambers 113 are conjugated to the impurities or the target biological substance in the sample, the magnetic beads may be attracted by the second magnet 1312 to separate the impurities or the target biological substance from the solution.

It should be noted that categorizing the magnets 131 as the first magnet 1311 and the second magnet 1312 is for illustrative purposes only and is intended to illustrate the positional relationship of the first magnet 1311 and the second magnet 1312 with a processing box 110, and the specific structures, materials, etc. of the first magnet 1311 and the second magnet 1312 are not limited. In some embodiments, the first magnet 1311 and the second magnet 1312 may be the same or similar. For example, the materials, dimensions, etc. of the first magnet 1311 and the second magnet 1312 are the same. In some embodiments, the first magnet 1311 and the second magnet 1312 may be different. In some embodiments, the magnet 131 may act as both the first magnet 1311 and the second magnet 1312. For example, in the embodiment shown in Fig. 3, the magnet 131 located on the right side of the right-most processing box 110 (which may be referred to as the first processing box) in the Y-axis direction may be referred to as the first magnet 1311, and the magnet 131 located on the left side of the first processing box may be referred to as the second magnet 1312, while for an intermediate processing box 110 (which may be referred to as the second processing box), the magnet 131 located on the left side of the first processing box may be referred to as the first magnet 1311.

In some embodiments, as shown in Figs. 3-5, the magnetic assembly 130 may further include a magnet driving mechanism 132. The magnet driving mechanism 132 may include a first driving motor 133, a lead screw 134 and a linkage 135. The lead screw 134 is drivingly connected to the first driving motor 133, one end of the linkage 135 (also called a first end) is hinged to the lead screw 134, the other end of the linkage 135 (also called a second end) is hinged to the magnet 131, and the magnet 131 can be driven by the first driving motor 133 to extend above or retract below an upper side surface of the magnetic attraction and mixing mechanism 120. The upper side surface of the magnetic attraction and mixing mechanism 120 may be a surface of the magnetic attraction and mixing mechanism 120 for carrying the processing box 110. For example, the mixing assembly 140 may include a mixing platform 142, and the processing box 110 may be disposed on an upper side surface of the mixing platform 142. After the magnet 131 extends above the upper side surface of the mixing platform 142, the magnetic beads in the processing box 110 can be attracted. When the magnet 131 is retracted below the upper side surface of the mixing platform 142, the magnetic beads in the processing box 110 are not affected by the magnet 131.

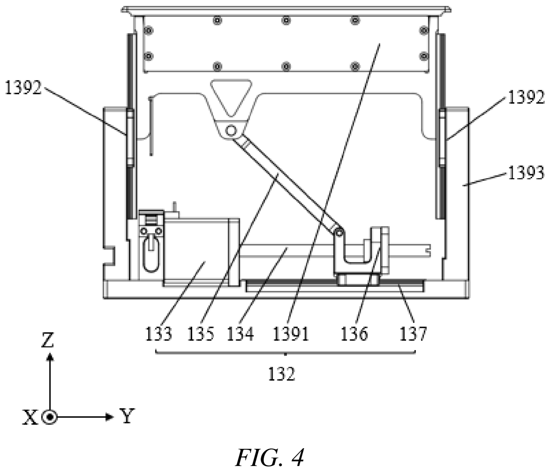

By way of example only, the magnet driving mechanism 132 may include a lead screw nut 136 and a nut track 137. The lead screw nut 136 is connected between the lead screw 134 and the linkage 135, and an extending direction of the nut track 137 is the first direction (i.e., the Y-axis direction). The lead screw nut 136 is connected to the end of the lead screw 134 away from the first driving motor 133, and the lead screw nut 136 is hinged to the first end of the linkage 135. The lead screw nut 136 may slide on the nut track 137. That is, the lead screw nut 136 can move in the first direction. The first driving motor 133 operates to drive the lead screw 134 to rotate, the rotation of the lead screw 134 drives the lead screw nut 136 to move in the first direction, and the movement of the lead screw nut 136 further drives the linkage 135 to rotate in the Y-Z plane, thereby driving the magnet 131 to perform a lifting or lowering movement in the vertical direction (i.e., the Z-axis direction). For example, when the lead screw nut 136 is moved to the right in Fig. 4, the linkage 135 is driven to rotate counterclockwise about an axis of rotation of the lead screw nut 136, thereby driving the magnet 131 to descend in the Z-axis direction, so that the magnet 131 gradually retracts below the upper side surface of the magnetic attraction and mixing mechanism 120. When the lead screw nut 136 is moved to the left in Fig. 4, the linkage 135 is driven to rotate clockwise about an axis of rotation of the lead screw nut 136, thereby driving the magnet 131 to ascend in the Z-axis direction, so that the magnet 131 gradually extends above the upper side surface of the magnetic attraction and mixing mechanism 120.

In some embodiments, the second end of the linkage 135 may be hinged directly to the magnet 131. For example, the second end of the linkage 135 and the magnet 131 may each be provided with a hinge, and the second end of the linkage 135 is hinged to the magnet 131 via the hinges. In some embodiments, the second end of the linkage 135 may be hinged indirectly to the magnet 131. For example, the magnet 131 may be disposed on a magnet support member 1391 of Fig. 4, and the second end of the linkage 135 is hinged to the magnet support member 1391.

In some embodiments, the magnetic assembly 130 may further include a magnet support member 1391 for fixing the magnet 131. In some embodiments, the magnet support member 1391 may be hinged to the linkage 135. The magnet 131 is fixed to the magnet support member 1391, thereby enabling indirect hinging of the second end of the linkage 135 to the magnet 131.

In some embodiments, an upper end of the magnet support member 1391 has a larger dimension in the Y-axis direction, while a lower end of the magnet support member 1391 has a smaller dimension in the Y-axis direction. The upper end of the magnet support member 1391 may be the end for supporting the magnet 131, and the lower end of the magnet support member 1391 may be the end hinged to the linkage 135. The larger dimension of the upper end makes the upper end easier to support the magnet 131, and the smaller dimension of the lower end makes the lower end easier to be hinged to the linkage 135.

In some embodiments, a linear guide rail 1392 may be provided on each side of the magnet support member 1391. An extending direction of the linear guide rail 1392 is the vertical direction (i.e., the Z-axis direction). The magnet support member 1391 can move in the vertical direction on the linear guide rail 1392. A linear guide rail 1392 is provided on each side of the magnet support member 1391, which can ensure that the magnet support member 1391 remains balanced when moving in the vertical direction. In some embodiments, the magnet support member 1391 may be connected to a base 1393 via the linear guide rails 1392, thereby enabling the magnet support member 1391 to move along the linear guide rails 1392 relative to the base 1393. The first driving motor 133 is located below the magnet support member 1391, and the lead screw nut 136 is connected to the magnet support member 1391 via the linkage 135. The first driving motor 133 operates to drive the lead screw 134 to rotate, the rotation of the lead screw 134 drives the lead screw nut 136 to move in the first direction, and the movement of the lead screw nut 136 further drives the linkage 135 to rotate in the Y-Z plane, thereby driving the magnet support member 1391 to move on the linear guide rails 1392 and achieving the lifting or lowering movement of the magnet 131.

In some embodiments, the extraction apparatus 10 may further include a magnet position sensor which may be configured to determine the position of the magnet 131 in the Z-axis direction, for example, to determine whether the magnet 131 is in a first position in the Z-axis direction (i.e., the magnet 131 fully extends above the upper side surface of the magnetic attraction and mixing mechanism 120) or in a second position in the Z-axis direction (i.e., the magnet 131 fully retracts below the upper side surface of the magnetic attraction and mixing mechanism 120). By way of example only, the magnet position sensor may include a first optocoupler and a first optocoupler barrier. One first optocoupler (not shown) is provided at each of the bottom and the top of the base 1393, and the first optocoupler barrier (not shown) is fixed to the magnet support member 1391. When the magnet support member 1391 moves along the linear guide rails 1392 to make the first optocoupler at the top of the base 1393 come into contact with the first optocoupler barrier, the first optocoupler barrier can generate a signal, telling that the magnet 131 is in the first position. When the magnet support member 1391 moves along the linear guide rails 1392 to make the first optocoupler at the bottom of the base 1393 come into contact with the first optocoupler barrier, the first optocoupler barrier can generate a signal, telling that the magnet 131 is in the second position.

It should be noted that in other embodiments, the magnet driving mechanism 132 may be configured to drive the magnet 131 to move in other directions, for example, in the X-axis direction and/or the Y-axis direction. In some embodiments, a plurality of magnet driving mechanisms 132 may be provided. The plurality of magnet driving mechanisms 132 are respectively used to drive the magnet 131 to move in different directions (e.g., the X-axis direction, the Y-axis direction, and the Z-axis direction).

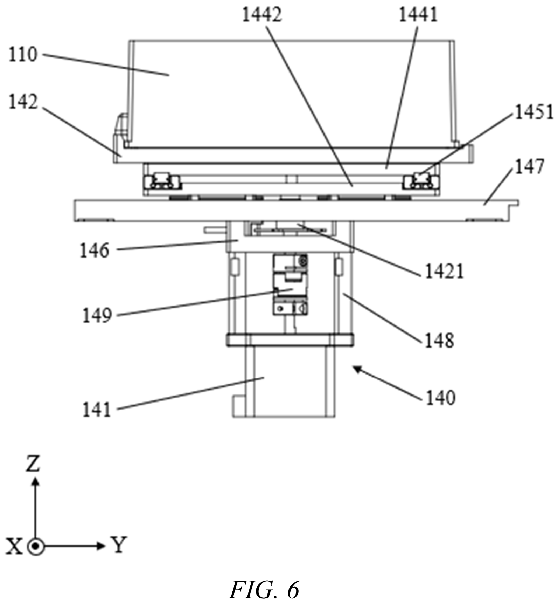

In some embodiments, the mixing assembly 140 may adopt an electric motor driving mode (e.g., electrical transmission driving or shaking table electromagnetic driving) or any other feasible driving modes (such as hydraulic driving, or pneumatic driving). In some embodiments, as shown in Figs. 6-8, the mixing assembly 140 may include a second driving motor 141 and a mixing platform 142. The mixing platform 142 may include an eccentric shaft 1421, and the second driving motor 141 may be drivingly connected to the eccentric shaft 1421. The mixing platform 142 may be configured to mount the processing box 110. When the processing box 110 is mounted on the mixing platform 142, the eccentric shaft 1421 may be driven by the second driving motor 141 to move, so as to drive the mixing platform 142 to shake or vibrate, thereby mixing the sample in the processing chambers 111.

By way of example only, the mixing assembly 140 may include a first mixing bearing 1431, a second mixing bearing 1432, a third mixing bearing 1433, a first direction adapter plate 1441, a second direction adapter plate 1442, a first mixing guide rail 1451, a second mixing guide rail 1452, a motor fixing plate 146, and a support plate 147. The first direction adapter plate 1441, the second direction adapter plate 1442, and the support plate 147 are parallel to one another. In the third direction (i.e., the Z-axis direction), the mixing platform 142 is located above the first direction adapter plate 1441, the second direction adapter plate 1442 is located below the first direction adapter plate 1441, and the support plate 147 is located below the second direction adapter plate 1442. The first direction adapter plate 1441 is connected to the second direction adapter plate 1442 via the first mixing guide rail 1451, and the second direction adapter plate 1442 is connected to the support plate 147 via the second mixing guide rail 1452. The first mixing guide rail 1451 may be perpendicular to the second mixing guide rail 1452, the first mixing guide rail 1451 may extend in the second direction (i.e., the X-axis direction), and the second mixing guide rail 1452 may extend in the first direction (i.e., the Y-axis direction). The motor fixing plate 146 may be connected to the underside of the support plate 147, and the second driving motor 141 may be connected to the motor fixing plate 146. One end of the eccentric shaft 1421 is connected to the second driving motor 141, and the other end thereof passes through the support plate 147 and the second direction adapter plate 1442 and is connected to the first direction adapter plate 1441 via the first mixing bearing 1431. The eccentric shaft 1421 is connected to the second direction adapter plate 1442 via the second mixing bearing 1432, and to the support plate 147 via the third mixing bearing 1433. When the second driving motor 141 drives the eccentric shaft 1421 to move, the eccentric shaft 1421 may drive the second direction adapter plate 1442 to move along the second mixing guide rail 1452 relative to the support plate 147, and drive the first direction adapter plate 1441 to move along the first mixing guide rail 1451 relative to the second direction adapter plate 1442, thereby driving the mixing platform 142 to vibrate in the first and second directions, to mix the sample.

In some embodiments, the second driving motor 141 may be directly fixed to the motor fixing plate 146. In some embodiments, the second driving motor 141 may be fixed to the motor fixing plate 146 via a connecting shaft 148. In some embodiments, the eccentric shaft 1421 may be directly connected to a rotary output shaft of the second driving motor 141. In some embodiments, the mixing assembly 140 may include a coupling 149, and the eccentric shaft 1421 may be connected to the rotary output shaft of the second driving motor 141 via the coupling 149.

In some embodiments, as shown in Figs. 6-8, the eccentric shaft 1421 is provided with a second optocoupler barrier 512, and the motor fixing plate 146 is provided with a second optocoupler 511. As the eccentric shaft 1421 rotates, the second optocoupler barrier 512 is driven to rotate. When the second optocoupler barrier 512 moves to the position of the second optocoupler 511, it can be determined that the eccentric shaft 1421 is in the initial position, and accordingly the processing box 110 is also in the initial position. In some embodiments, the processing box 110 in the initial position is closer to the magnet 131. In some cases, determining whether the processing box 110 is in the initial position by providing the second optocoupler barrier 512 and the second optocoupler 511 can, on the one hand, assist in positioning the mechanical arm device 400 when aspirating liquid from the processing box 110 and, on the other hand, place the processing box 110 in the initial position so that the processing box is closer to the magnet 131 when magnetic attraction is required.

In some embodiments, the number of sample processing devices 100 may be one or plural. In some embodiments, the number of processing boxes 110 may be one or plural, and the number of mixing assemblies 140 and the number of magnetic assemblies 130 may be adapted to the number of processing boxes 110. By way of example only, one processing box 110 may be subjected to mixing by means of one separate mixing assembly 140 and subjected to magnetic attraction by means of two separate magnetic assemblies 130, respectively. One of the magnetic assemblies 130 is configured to drive the first magnet 1311 to extend above or retract below the upper side surface of the magnetic attraction and mixing mechanism 120, and the other magnetic assembly 130 is configured to drive the second magnet 1312 to extend above or retract below the upper side surface of the magnetic attraction and mixing mechanism 120. That is, the number of processing boxes 110 is n, the number of mixing assemblies 140 is n, and the number of magnetic assemblies 130 is 2n. In another example, as shown in Fig. 3, the number of processing boxes 110 is 3, the number of mixing assemblies 140 is 3, the number of magnetic assemblies 130 is 4, the three processing boxes 110 are spaced apart in the X-axis direction, one mixing assembly 140 corresponds to one processing box 110, and the processing boxes 110 are respectively disposed on the mixing platforms (e.g., the mixing platforms 142 in Fig. 6) of the corresponding mixing assemblies 140, the four magnetic assemblies 130 are spaced apart from the processing boxes 110 in the X-axis direction, and two adjacent processing boxes 110 share the same magnetic assembly 130, to reduce the number of magnetic assemblies 130 and reduce the cost of the apparatus. In some embodiments, a plurality of processing boxes 110 may be subjected to mixing by means of the same mixing assembly 140.

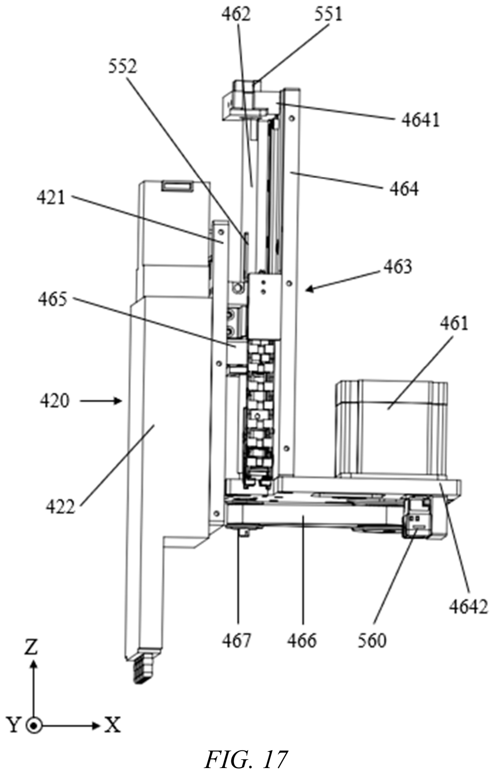

In some embodiments, as shown in Fig. 9, the consumable assembly 200 may include a reagent kit 210 and a pipette tip box 220. The reagent kit 210 may include one or more cavities 211 for containing reagents, and the pipette tip box 220 may include one or more pipette tips 221. The pipette tip 221 may mate with the mechanical arm device (e.g., a liquid pump 422 in Fig. 17) to meet the requirements of aspirating different reagents, magnetic beads and/or waste liquid during sample processing.

In some embodiments, the consumable assembly 200 may include a consumable support 230. The consumable support 230 may be configured to carry the consumable assembly 200. Before the processing of the sample, the reagent kit 210 and the pipette tip box 220 may be loaded onto the consumable support 230 according to the requirements of the sample processing process, to facilitate the addition of a reagent required for processing to the sample during the processing of the sample, and to facilitate the mating of the operating arm 420 with the pipette tip 221. In some embodiments, the reagent kit 210 and the pipette tip box 220 may be loaded onto the consumable support 230 either manually or automatically.

In some cases, individual consumables may be positioned by providing the consumable support. For example, individual consumables may be disposed on the consumable support in a preset arrangement to effectively reduce the overall space size of the extraction apparatus. Furthermore, the preset arrangement enables the individual consumables to be adapted to the set of guide rails, thus optimizing the movement path of the operating arm, reducing the time spent on the movement of the operating arm, and improving the sample processing efficiency.

In some embodiments, when the reagent kit 210 includes a plurality of cavities 211, the plurality of cavities 211 may be arrayed in one or more rows (e.g., 1 row, 2 rows, or 3 rows) in the X-axis direction (i.e., the second direction). Each row of cavities 211 extends in the Y-axis direction (or the first direction). In some cases, the arrangement of the plurality of cavities 211 in a plurality of rows can prevent the length of the reagent kit 210 along the Y axis from being too long due to the excessive number of cavities 211 required, so that the overall length of the extraction apparatus 10 in the Y-axis direction can be prevented from being too long. As shown in Fig. 9, the reagent kit 210 includes a plurality of cavities 211 arranged in four rows. In some embodiments, the volumes of the plurality of cavities 211 may be the same or different. For example, the cavities 211 may be sized according to the amounts of reagents to be contained. The cavities 211 may respectively contain the same or different reagents. For example, a corresponding type of reagent may be selected according to the target biological substance to be extracted and/or the processing process.

In some embodiments, the head of the pipette tip 221 for aspirating liquid may face the interior of the pipette tip box 220, and the tail of the pipette tip 221 that cooperates with the mechanical arm device 400 faces the outside of the pipette tip box 220. In some embodiments, the pipette tip 221 may be disposed vertically or approximately vertically, so as to facilitate the mating of the tail of the pipette tip 221 with the mechanical arm device 400. In some embodiments, the plurality of pipette tips 221 may or may not be exactly the same. For example, the pipette tip 221 may be sized according to the amount of liquid and/or magnetic beads to be pipetted by the pipette tip 221. For another example, the pipette tips 221 with different structures may be provided to adapt to different reagents, facilitating the smooth aspiration of each reagent. In some embodiments, a single pipette tip 221 may be used once or repeatedly during the processing of the sample. For example, at two stages during the processing of the sample where the same reagent needs to be pipetted from the reagent kit 210, the same pipette tip 221 may be repeatedly used to pipette the same reagent. For another example, in order to make the amount of pipetted reagent more accurate or avoid mutual contamination, each pipette tip 221 is only used to pipette the liquid and/or magnetic beads once.

In some embodiments, the consumable assembly 200 may further include a target biological substance storage box 240. The target biological substance storage box 240 is configured to contain a target biological substance obtained by means of extraction, separation and/or purification during the processing of the sample. Before the processing of the sample, one or more target biological substance storage boxes 240 may be loaded onto the consumable support 230 according to the requirements of the sample processing process to facilitate the storage of the target biological substance.

In some embodiments, the target biological substance storage box 240 is divided into a plurality of chambers 241 by means of partitions. The plurality of chambers 241 may be arrayed in one or more rows (e.g., 1 row, 2 rows, or 3 rows) in the X-axis direction (i.e., the second direction). Each row of chambers 241 extends in the Y-axis direction (i.e., the first direction). In some embodiments, the target biological substance storage box 240 may include one or more target biological substance collection tubes (not shown). For example, the number and/or the size of target biological substance collection tubes may be determined according to the amount of target biological substances that can be collected. In some embodiments, the target biological substance collection tube 3026 is loaded in the chamber 241 of the target biological substance storage box 240.

In some embodiments, the pipette tips 221 may correspond to the chambers 241 of the target biological substance storage box 240 on a one-to-one basis. That is, one pipette tip 221 corresponds to one chamber 241 of the target biological substance storage box 240 to facilitate the bulk transfer of target biological substances into the chambers 241 of the target biological substance storage box 240 using the pipette tips 221. By way of example only, as shown in Fig. 9, the number of rows of pipette tips 221 and the number of rows of chambers 241 of the target biological substance storage box 240 may be the same. The number of pipette tips 221 per row may be the same as the number of chambers 241 per row of the target biological substance storage box 240.

In some embodiments, the consumable assembly 200 may further include a sample preprocessing box 250 and a magnetic bead storage box 260. The sample preprocessing box 250 is a container for sample containing, sample preprocessing, and/or sample reaction. The magnetic bead storage box 260 may be configured to contain the magnetic beads used during the processing of the sample. In some embodiments, the sample preprocessing may include biological culture, resuspension, magnetic attraction, separation, or any other feasible biological preprocessing operations. In some embodiments, the sample reaction may include resuspension, magnetic attraction, separation, lysis, precipitation or any other feasible sample reaction operations.

In some embodiments, the magnetic bead storage box 260 may include one or more holding chambers 261 for containing magnetic beads. The number of holding chambers 261 may vary depending on the type of magnetic beads, and different magnetic beads may be placed in different holding chambers 261. For example, as shown in Fig. 9, the magnetic bead storage box 260 may include two holding chambers 261. One of the holding chambers 261 is configured to hold first magnetic beads, and the other holding chamber 261 is configured to hold second magnetic beads.

In some embodiments, the consumable assembly 200 may further include a waste liquid box 270. The waste liquid box 270 is provided with a waste liquid discharge channel (not shown). The waste liquid box 270 may be configured to hold waste liquid produced during the processing of the sample. In some embodiments, the waste liquid box 270 is removably disposed on the consumable support 230 to facilitate waste liquid collection. In some embodiments, the waste liquid box 270 is fixed relative to the consumable support 230. In some embodiments, the waste liquid box 270 is provided with a waste liquid discharge channel (not shown) which may communicate the waste liquid box 270 with an external waste liquid storage tank (not shown) to prevent excessive waste liquid in the waste liquid box 270 from overflowing.

It should be noted that the description of the consumable assembly in the present description is for illustrative purposes only and that the layout position of each consumable can be adjusted and moved as required. For example, in Fig. 9, the target biological substance storage box 240 may be interchanged with one of the pipette tip boxes 220. Since the layout position of each consumable in the present description can be adjusted as required, the layout of the consumable assembly is flexible and adaptable to a variety of application scenarios.

In some embodiments, as shown in Figs. 10-11, the sample preprocessing box 250 and the magnetic bead storage box 260 are disposed on the mixing mechanism 300, and the mixing mechanism 300 may preprocess the sample in the sample preprocessing box 250 (e.g., mix the supernatant and sediment into a resuspended solution), and shake the magnetic beads in the magnetic bead storage box 260 to prevent the magnetic beads from settling.

In some embodiments, the sample preprocessing box 250 and the magnetic bead storage box 260 may be configured to be separately subjected to mixing by means of different mixing mechanisms 300. In some embodiments, the sample preprocessing box 250 and the magnetic bead storage box 260 may be configured to be subjected to mixing by means of the same mixing mechanism 300.

In some embodiments, the mixing mechanism 130 may adopt an electric motor driving mode (e.g., electrical transmission driving or shaking table electromagnetic driving) or any other feasible driving modes (such as hydraulic driving or pneumatic driving). In some embodiments, the mixing mechanism 300 may include a third driving motor 310 and a second mixing platform 320. The second mixing platform 320 may include a second eccentric shaft 321, and the third driving motor 310 may be drivingly connected to the second eccentric shaft 321. The second mixing platform 320 may be configured to mount the sample preprocessing box 250 and/or the magnetic bead storage box 260. When the sample preprocessing box 250 and/or the magnetic bead storage box 260 are mounted on the second mixing platform 320, the second eccentric shaft 321 may be driven by the third driving motor 310 to move, so as to drive the second mixing platform 320 to shake or vibrate. By way of example only, the mixing mechanism 300 may include a fourth mixing bearing 331, a fifth mixing bearing 332, a sixth mixing bearing 333, a third direction adapter plate 341, a fourth direction adapter plate 342, a third mixing guide rail 351, a fourth mixing guide rail 352, a second motor fixing plate 360, a second support 370, and a second base 380. The third direction adapter plate 341, the fourth direction adapter plate 342, and the second motor fixing plate 360 are parallel to one another. In the third direction (i.e., the Z-axis direction), the second mixing platform 320 is located above the third direction adapter plate 341, the fourth direction adapter plate 342 is located below the third direction adapter plate 341, and the second motor fixing plate 360 is located below the fourth direction adapter plate 342. The third direction adapter plate 341 is connected to the fourth direction adapter plate 342 via the third mixing guide rail 351, and the fourth direction adapter plate 342 is connected to the second support plate 370 via the fourth mixing guide rail 352. The third mixing guide rail 351 may be perpendicular to the fourth mixing guide rail 352, the third mixing guide rail 351 may extend in the second direction (i.e., the X-axis direction), and the third mixing guide rail 351 may extend in the first direction (i.e., the Y-axis direction). The third driving motor 310 may be connected to the second support 370 via the second motor fixing plate 360, and then to the second base 380 via the second support 370. One end of the second eccentric shaft 321 is connected to the third driving motor 310, and the other end thereof passes through the second motor fixing plate 360 and the fourth direction adapter plate 342 and is connected to the third direction adapter plate 341 via the fourth mixing bearing 331. The second eccentric shaft 321 is connected to the fourth direction adapter plate 342 via the fifth mixing bearing 332, and to the second motor fixing plate 360 via the sixth mixing bearing 333. When the third driving motor 310 drives the second eccentric shaft 321 to move, the second eccentric shaft 321 may drive the fourth direction adapter plate 342 to move along the fourth mixing guide rail 352 relative to the second support plate 370, and drive the third direction adapter plate 341 to move along the third mixing guide rail 351 relative to the fourth direction adapter plate 342, thereby driving the second mixing platform 320 to vibrate in the first and second directions, to mix the sample in the sample preprocessing box 250 and the magnetic beads in the magnetic bead storage box 260.

In some embodiments, the third driving motor 310 may be directly fixed to the second motor fixing plate 360. In some embodiments, the third driving motor 310 may be fixed to the second motor fixing plate 360 via a second connecting shaft (not shown). In some embodiments, the second eccentric shaft 321 may be directly connected to a rotary output shaft of the third driving motor 310. In some embodiments, the mixing mechanism 300 may further include a second coupling (not shown) via which the second eccentric shaft 321 may be connected to the rotary output shaft of the third driving motor 310.

In some embodiments, an angle of rotation of the second eccentric shaft 321 may be determined by providing a third optocoupler 521 and a third optocoupler barrier 522. The third optocoupler 521 and the third optocoupler barrier 522 may cooperate in the same or similar manner as the second optocoupler 511 and the second optocoupler barrier 512, and will not be described here.

In some embodiments, as shown in Figs. 12-18, the number of first guide rails 411 is two. The two first guide rails 411 are disposed opposite each other, and the second guide rail 412 is disposed between the two first guide rails 411. In some embodiments, the mechanical arm device 400 may further include a support frame 430. The support frame 430 may be configured to carry the set of guide rails 410 and the operating arm 420. In some embodiments, the two first guide rails 411 may be disposed oppositely on two sides of the support frame 430 in the Y-axis direction, and the extending direction of the two first guide rails 411 is parallel to the X-axis direction. The extending direction of the second guide rail 412 is parallel to the Y-axis direction, the second guide rail 412 is movable along the first guide rails 411, and the operating arm 420 is movably disposed on the second guide rail 412. In this embodiment, since the second guide rail 412 is movable along the first guide rails 411 and the operating arm 420 is movably disposed on the second guide rail 412, the operating arm 420 can be made to move in the X-axis direction by controlling the movement of the second guide rail 412 along the first guide rails 411, and the operating arm 420 can be made to move in the Y-axis direction by controlling the movement of the operating arm 420 along the second guide rail 412, so that the operating arm 420 can move between the sample processing device 100 and the consumable assembly 200 during the processing of the sample.

In some embodiments, as shown in Figs. 12-14, the mechanical arm device 400 may further include a first synchronizing belt 441, a first pulley 444, and a first guide rail motor 442. The first synchronizing belt 441 may be disposed along the first guide rail 411, the first pulley may be fixed at one end of the first synchronizing belt, the first guide rail motor 442 may drive the first pulley 444 to rotate, and the first pulley 444 may drive the first synchronizing belt 441 to move. The second guide rail 412 can be driven by the first synchronizing belt 441 to move in a direction perpendicular to the first guide rail 411.

By way of example only, the mechanical arm device 400 may further include a drive shaft 443, and the two first guide rails 411 each have one end connected to the support frame 430 and the other end each provided with the first pulley 444. The drive shaft 443 is disposed on the support frame 430, and the extending direction of the drive shaft 443 is parallel to the extending direction of the second guide rail 412. The second guide rail 412 is provided at each end with the first synchronizing belt 441, and the first guide rail motor 442 is disposed at one end (which may be referred to as a first end) of the drive shaft 443. A rotary output shaft of the first guide rail motor 442, a first end of the drive shaft 443 and the first pulley 444 on one of the first guide rails 411 are drivingly connected via the first synchronizing belt 441, and the first synchronizing belt 441 is fixedly connected to one end (which may be referred to as a first end) of the second guide rail 412. A second end of the drive shaft 443 and the first pulley 444 on the other first guide rail 411 are drivingly connected via the other first synchronizing belt 441, which is fixedly connected to the other end (which may be referred to as a second end) of the second guide rail 412. When the rotary output shaft of the first guide rail motor 442 rotates, it is possible to drive the first synchronizing belt 441 connected thereto to move, then drive the first end of the second guide rail 412 to move, and drive the drive shaft 443 to rotate. The drive shaft 443 then may drive the second end of the second guide rail 412 to move such that the two ends of the second guide rail 412 are moved synchronously, and finally moved in the direction perpendicular to the first guide rail 411 (i.e., in the X-axis direction).

In some embodiments, as shown in Figs. 1 and 15-16, the mechanical arm device 400 may further include a second synchronizing belt 451, a second pulley 458 and a second guide rail motor 452. The second guide rail motor 452 is disposed on the operating arm 420, the second synchronizing belt 451 may be disposed along the second guide rail 412, and the second pulley 458 may be fixed to the operating arm 420 and can move along the second synchronizing belt 451. The second guide rail motor 452 may drive the second pulley 458 to rotate such that the second pulley 458 is moved along the second synchronizing belt 451, and the operating arm 420 may move along the second guide rail 412 with the second pulley 458.

By way of example only, the mechanical arm device 400 may include a crossbeam 453, a second guide rail support plate 454, and a second guide rail slider 455. The crossbeam 453 is configured to extend in the Y-axis direction, the second guide rail support plate 454 is fixed to the crossbeam 453, and the second guide rail 412 is disposed on the second guide rail support plate 454. The second guide rail slider 455 is movably disposed on the second guide rail 412, and the second guide rail slider 455 is provided with a second motor support plate 456 and the operating arm 420. The second motor support plate 456 is provided with a second guide rail motor 452 and two idlers 457. The two idlers 457 are distributed on two sides of the second guide rail motor 452 in the Y-axis direction. The second pulley 458 is disposed on the second guide rail slider 455 coaxially with a rotary output shaft of the second guide rail motor 452, the second pulley 458 and the two idlers 457 are axially parallel to each other, the second synchronizing belt 451 is disposed on the second guide rail support plate 454, and the second pulley 458 and the two idlers 457 are drivingly connected via the second synchronizing belt 451. When the rotary output shaft of the second guide rail motor 452 rotates, it is possible to drive the second pulley 458 to rotate, then drive the second guide rail slider 455 to move by means of the second synchronizing belt 451, and finally move the operating arm 420 in the Y-axis direction.

In some embodiments, as shown in Figs. 1 and 17-18, the mechanical arm device 400 may further include a lifting motor 461 and a lifting lead screw 462. The operating arm 420 is connected to the lifting lead screw 462, and the lifting motor 461 drives the lifting lead screw 462 to move and drives the operating arm 420 to perform a lifting or lowering movement (i.e., a movement in a direction perpendicular to the horizontal plane).

By way of example only, the mechanical arm device 400 may include a lifting guide rail 463, a lifting guide rail support plate 464, a lifting guide rail slider 465, and a third synchronizing belt 466. The lifting guide rail support plate 464 is disposed along the Z-axis direction, the lifting guide rail 463 is disposed on the lifting guide rail support plate 464, and the lifting guide rail support plate 464 is connected to the second guide rail slider 455. Top and bottom ends of the lifting guide rail support plate 464 are provided with a top support plate 4641 and a bottom support plate 4642, respectively, and the lifting motor 461 is disposed on the bottom support plate 4642. The lifting lead screw 462 and the lifting nut 467 that cooperates with the lifting lead screw 462 are connected to the top support plate 4641 and the bottom support plate 4642, respectively, via bearings. The lifting nut 467 is drivingly connected to a rotary output shaft of the lifting motor 461 via the third synchronizing belt 466. The lifting guide rail slider 465 is movably disposed on the lifting guide rail 463 and is connected to the lifting lead screw 462. The operating arm 420 may include a pump connecting plate 421 and a liquid pump 422. The liquid pump 422 is connected to the lifting guide rail slider 465 via the pump connecting plate 421, and the liquid pump 422 may be configured to mate with a pipette tip (e.g., the pipette tip 221 in Fig. 9). When the rotary output shaft of the lifting motor 461 rotates, it is possible to drive the lifting nut 467 to move by means of the third synchronizing belt 466, then drive the lifting lead screw 462 and the lifting guide rail slider 465 to move along the lifting guide rail 463, and finally realize the lifting or lowering movement of the operating arm 420 in the Z-axis direction.

In some embodiments, the first guide rail 411 and second guide rail 412 are respectively provided with position sensors for detecting the position of the operating arm 420 in the X-axis direction and the Y-axis direction, respectively. In some embodiments, the position sensor may be at least one of the following types of sensors: a photoelectric sensor, a Hall sensor, and a laser sensor.

In some embodiments, the first end of the second guide rail 412 is provided with a fourth optocoupler barrier 532, and an end of the first guide rail 411 connected to the first end of the second guide rail 412 is provided with a fourth optocoupler 531. When the fourth optocoupler barrier 532 is moved to the fourth optocoupler 531, the fourth optocoupler 531 may generate a signal. In this case, it can be determined that the second guide rail 412 and the operating arm 420 are in their initial positions in the X-axis direction.

In some embodiments, one end of the crossbeam 453 is provided with a fifth optocoupler 541, and the second motor support plate 456 is provided with a fifth optocoupler barrier 542. When the fifth optocoupler barrier 542 is moved to the fifth optocoupler 541, the fifth optocoupler 541 may generate a signal. In this case, it can be determined that the second guide rail slider 455 and the operating arm 420 are in their initial positions in the Y-axis direction.

In some embodiments, the top support plate 4641 is provided with a sixth optocoupler 551, and the lifting guide rail slider 465 is provided with a sixth optocoupler barrier 552. When the sixth optocoupler barrier 552 is moved to the sixth optocoupler 551, the sixth optocoupler 551 may generate a signal. In this case, it can be determined that the operating arm 420 is in its initial position in the Z-axis direction.

In some embodiments, a scanning sensor 560 is provided below the bottom support plate 4642. The scanning sensor 560 may be configured to scan whether there are contents in a reagent kit (e.g., the reagent kit 210 in Fig. 9), in a pipette tip box (e.g., the pipette tip box 220 in Fig. 9), and in a target biological substance storage box (e.g., the target biological substance storage box 240 in Fig. 9) during the movement of the operating arm 420. By way of example, the scanning sensor 560 may include an infrared sensor, a structural sensor, etc.

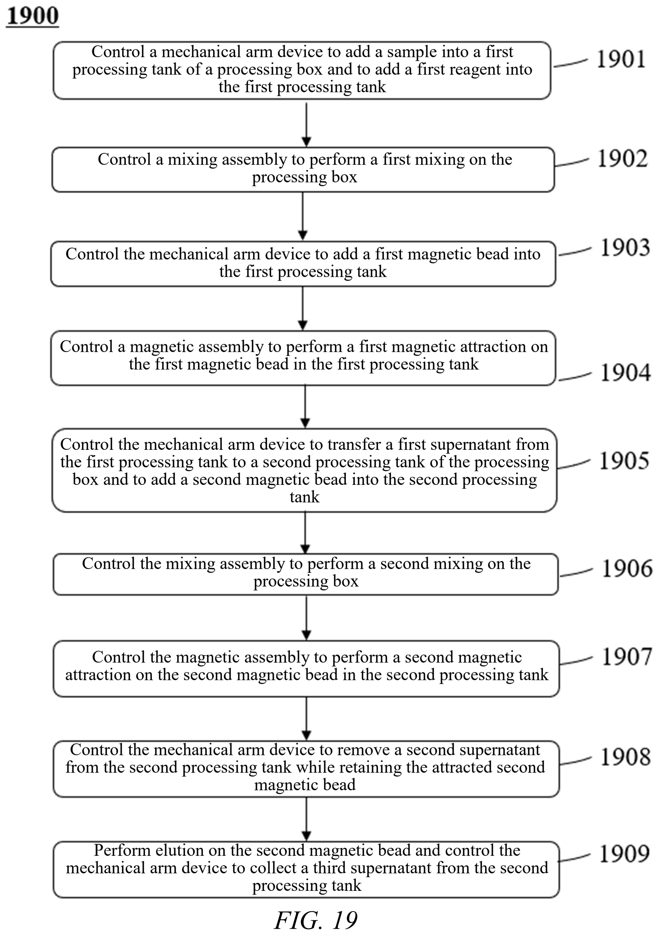

In some embodiments, the extraction apparatus described in the description of the present application may be applied to extract and/or purify a target biological substance. A method of use for the extraction apparatus described in the present description will be provided to extract, separate and/or purify a target biological substance. Before the extraction apparatus is started, the reagent kit, the pipette tip box, the target biological substance storage box, and the waste liquid box may be loaded onto the consumable support, the magnetic bead box and the sample preprocessing box may be loaded onto the second mixing platform, and the processing box may be loaded onto the mixing platform. In some embodiments, the loading process may be performed manually by an experimenter, or by a robot (e.g., a mechanical arm). In some embodiments, the sample in the sample preprocessing box may be preprocessed. After loading is completed, the extraction apparatus may be started to automatically extract and/or purify the target biological substance. In some embodiments, as shown in Fig. 19, a method of use 1900 of an extraction apparatus may include the following steps.

In step 1901, a mechanical arm device is controlled to add a sample into a first processing chamber of a processing box and to add a first reagent into the first processing chamber.

In step 1902, a mixing assembly is controlled to perform a first mixing on the processing box.

In some embodiments, it is necessary to perform mixing on the first processing chamber after the first reagent is added in order for the sample to react fully with the first reagent. In some embodiments, the second driving motor may be controlled to rotate at a first preset rotation speed for a first preset period of time to mix the first reagent with the sample. In some embodiments, when the first reagent includes two or more types, different first reagents may be added in sequence. For example, the first reagent may include a first sub-reagent and a second sub-reagent, the first preset rotation speed may comprise a first preset sub-rotation speed and a second preset sub-rotation speed, and the first preset period of time may comprise a first preset sub-period of time and a second preset sub-period of time. After the first sub-reagent is added, the second driving motor may be controlled to rotate at the first preset sub-rotation speed for the first preset sub-period of time to mix the first sub-reagent with the sample. The second sub-reagent is then added after the first preset period of time expires, and the second driving motor is controlled to rotate at the second preset sub-rotation speed for the second preset sub-period of time to mix the second sub-reagent with the sample mixed with the first sub-reagent. In some embodiments, both the first sub-reagent and the second sub-reagent may refer to reagents that can undergo a lysis reaction with a sample or a solution. In some embodiments, the type and the volume of the first reagent added may be adjusted according to actual requirements.

In step 1903, the mechanical arm device is controlled to add first magnetic beads into the first processing chamber.

In some embodiments, the first magnetic beads may be configured to be conjugated to the sample mixed with the first reagent or impurities in a solution. In some embodiments, in order to improve the attraction effect of the first magnetic beads on the impurities and the attraction rate, during the attraction process, the mixing assembly may be controlled to perform the first mixing on the processing box. In some embodiments, the first mixing may be to control the second driving motor to rotate at a second preset rotation speed for a second preset period of time such that the first magnetic beads are fully conjugated to the impurities.

In step 1904, a magnetic assembly is controlled to perform a first magnetic attraction on the first magnetic beads in the first processing chamber.

In some embodiments, the first magnetic attraction may be to control a first magnet to extend above an upper side surface of a mixing platform by controlling a first driving motor, to use a magnetic force of the first magnet to attract the first magnetic beads to a side wall of the first processing chamber, and to stand for a third preset period of time. In some embodiments, the third preset period of time may be a period of time during which the extraction apparatus stops operation. In some embodiments, the third preset period of time may be set manually according to the extraction and purification process, or be set by default in the extraction apparatus. In some embodiments, the magnetic assembly may maintain magnetic attraction to the first magnetic beads in the first processing chamber within the third preset period of time. The supernatant may refer to a transparent liquid above a sediment layer (e.g., a first magnetic bead conjugate) in the processing box. For example, when the first magnetic beads are configured to be conjugated to the impurities, the first supernatant mainly contains the target biological substance to be extracted and a small amount of impurities.

In step 1905, the mechanical arm device is controlled to transfer a first supernatant from the first processing chamber to a second processing chamber of the processing box and to add second magnetic beads into the second processing chamber.

In some embodiments, the first magnetic bead and the second magnetic bead are the same. For example, objects to be conjugated by the second magnetic beads and the first magnetic beads are consistent. For example, both the first magnetic beads and the second magnetic beads are configured to be conjugated to the impurities. In some embodiments, the second magnetic bead is different from the first magnetic bead. For example, the first magnetic beads are configured to be conjugated to the impurities, while the second magnetic beads are configured to be conjugated to the target biological substance. In this embodiment, the second magnetic beads may be conjugated to the target biological substance. In some embodiments, prior to step 1905, steps 1903-1904 may be repeated one or more times (e.g., twice), to remove the impurities sufficiently.

In step 1906, a mixing assembly is controlled to perform a second mixing on the processing box.

In some embodiments, the second mixing process may refer to controlling a second driving motor to rotate at a third preset rotation speed for a fourth preset period of time to fully conjugate the second magnetic beads to the target biological substance.

In step 1907, the magnetic assembly is controlled to perform a second magnetic attraction on the second magnetic beads in the second processing chamber.

In some embodiments, the second magnetic attraction may be to control a second magnet to extend above an upper side surface of a mixing platform by controlling the first driving motor, to use a magnetic force of the second magnet to attract the second magnetic beads conjugated to the target biological substance to a side wall of the second processing chamber, and to stand for a fifth preset period of time. In some embodiments, the fifth preset period of time may be a period of time during which the extraction apparatus stops operation. In some embodiments, the fifth preset period of time may be set manually according to the extraction and purification process, or be set by default in the extraction apparatus. In some embodiments, the magnetic assembly may maintain magnetic attraction to the second magnetic beads in the second processing chamber within the fifth preset period of time.

In step 1908, the mechanical arm device is controlled to remove a second supernatant from the second processing chamber while retaining the attracted second magnetic beads.

In some embodiments, the second supernatant in the second processing chamber may be regarded as waste liquid when the second magnetic beads are configured to be conjugated to the target biological substance.

In step 1909, elution is performed on the second magnetic beads, and the mechanical arm device is controlled to collect a third supernatant from the second processing chamber.

In some embodiments, the step of performing elution on the second magnetic beads and controlling the mechanical arm device to collect a third supernatant from the second processing chamber may include the following steps. In step a, the magnetic assembly is controlled to drive the second magnet to retract below an upper side surface of a mixing platform, and the mechanical arm device is controlled to add a second reagent into the second processing chamber, the second reagent being capable of eluting a target biological substance from the second magnetic beads. In step b, the mixing assembly is controlled to drive the processing box to perform a third mixing, which may be similar to the principle of the first mixing or the second mixing, and will not be described here. In step c, the magnetic assembly is controlled to perform a third magnetic attraction on the second magnetic beads in the second processing chamber, and stand for a sixth preset period of time. The third magnetic attraction may be similar to the principle of the first magnetic attraction or the second magnetic attraction, and will not be described here. In step d, the mechanical arm device is controlled to collect the third supernatant from the second processing chamber. In some embodiments, after the second reagent is added, the second magnetic beads are separated from a second magnetic bead conjugate (e.g., the target biological substance), so that the third supernatant may be regarded as the target biological substance to be pipetted and/or purified. In some embodiments, the sixth preset period of time may be a period of time during which the extraction apparatus stops operation. In some embodiments, the sixth preset period of time may be set manually according to the extraction and purification process, or be set by default in the extraction apparatus. In some embodiments, the second magnet may maintain magnetic attraction to the second magnetic beads in the second processing chamber within the sixth preset period of time.

In some embodiments, prior to step a, steps 1905-1908 may be repeated one or more times (e.g., twice), to remove the impurities sufficiently.

In some embodiments, before aspiration, transfer, addition, and removal of the liquid, the magnetic beads and/or the reagent, the mechanical arm device may be controlled to mate with a pipette tip. By way of example only, when carrying out a liquid aspiration operation, the following steps may be included. After the assembly of the pipette tip, a first guide rail motor is controlled to move an operating arm in the X-axis direction (i.e., the second direction) and/or a second guide rail motor is controlled to move the operating arm in the Y-axis direction (i.e., the first direction) to a position where the liquid aspiration operation is required. The head of the pipette tip is positioned above this position. A lifting motor is controlled to move the operating arm downward in the Z-axis direction (i.e., the third direction) until the pipette tip comes into contact with the solution, and a liquid pump is controlled to perform the liquid aspiration operation.

In some embodiments, the assembly of the pipette tip may include the following steps. The first guide rail motor is controlled to move the operating arm in the X-axis direction (i.e., the second direction) and/or the second guide rail motor is controlled to move the operating arm in the Y-axis direction (i.e., the first direction) to a pipette tip box, such that the operating arm is located above the pipette tip to be assembled. The lifting motor is controlled to move the operating arm downward in the Z-axis direction (i.e., the third direction) until a suction head of the operating arm comes into contact with and is sealingly connected to the tail of the pipette tip. The operating arm is moved upward in the Z-axis direction (i.e., the third direction) to remove the head of the pipette tip from the pipette tip box.

In some embodiments, when a liquid discharge operation is performed, the liquid discharge step includes the following steps. The first guide rail motor is controlled to move the operating arm in the X-axis direction (i.e., the second direction) and/or the second guide rail motor is controlled to move the operating arm in the Y-axis direction (i.e., the first direction) to a position where the liquid discharge operation is required, such that the operating arm is located above that position. The lifting motor is controlled to move the operating arm downward in the Z-axis direction (i.e., the third direction) until the head of the pipette tip comes into contact with the solution. The liquid pump is controlled to perform the liquid discharge operation.

In some embodiments, the first supernatant is waste liquid when the first magnetic beads are configured to be conjugated to the target biological substance. The method of use of an extraction apparatus may further include the following steps. After the mechanical arm device is controlled to remove the first supernatant from the first processing chamber while retaining the attracted first magnetic beads, the magnetic assembly is controlled to stop the magnetic attraction. The mechanical arm device is controlled to add a fourth reagent into the first processing chamber for eluting the target biological substance from the first magnetic beads. The mixing assembly is controlled to perform mixing on the first processing chamber. The magnetic assembly is controlled to perform magnetic attraction on the mixed liquid in the first processing chamber. The mechanical arm device is controlled to collect a fourth supernatant from the first processing chamber, i.e. the target biological substance to be extracted and/or purified.

The extraction apparatus and the control method of the extraction apparatus provided in the present description may include, but not limited to, the following beneficial effects. (1) The sample processing device of the extraction apparatus provided in the present description can attract the magnetic beads in a sample that are conjugated to the impurities or the target biological substance, thereby facilitating the separation of the impurities or the target biological substance from the solution by means of the mechanical arm device. In addition, the extraction apparatus can mix the reagent and/or the magnetic beads added into the sample to improve the efficiency and effectiveness of the extraction and/or purification of the target biological substance. (2) Since the operating arm in the present description can not only move in the horizontal plane along the first guide rail and the second guide rail, but also extend and retract in the direction perpendicular to the horizontal plane, the operating arm can move between the sample processing device and the consumable assembly along the set of guide rails, which makes it possible to perform such operations as liquid transfer, waste liquid collection, pipette tip mating, and magnetic bead addition more efficiently and conveniently. (3) Driven by the magnet driving mechanism, the magnet can retract below the upper side surface of the magnetic attraction and mixing mechanism when there is no need to perform magnetic attraction on the biological substance, and extend above the upper side surface of the magnetic attraction and mixing mechanism when there is a need to perform magnetic attraction on the biological substance, so that the processing device can meet the various requirements of the different steps of extraction and/or purification of the biological substance, thereby improving the applicability. (4) In order to prevent the biological substance in the other set of processing chambers from being affected, only one of the two sets of processing chambers is used to process the sample at the same moment.

The above is merely preferred embodiments of the present description, which, however, are not intended to limit the present description, and any modifications, equivalent replacements, improvements, etc. made within the spirit and principle of the present description should fall within the scope of protection of the present description.

## Claims

1. An extraction apparatus for a sample, **characterized by** comprising:
a sample processing device, comprising a magnetic attraction and mixing mechanism for performing mixing and magnetic attraction on a sample;
a mechanical arm device, comprising a set of guide rails and an operating arm movable along the set of guide rails, the set of guide rails comprising a first guide rail and a second guide rail perpendicular to each other in a horizontal plane, and the operating arm being movable in a direction perpendicular to the horizontal plane; and
a consumable assembly, wherein the operating arm is movable between the sample processing device and the consumable assembly along the set of guide rails.

2. The extraction apparatus according to claim 1, **characterized by** further comprising a mixing mechanism, wherein the consumable assembly comprises a sample preprocessing box and a magnetic bead storage box which are disposed on the mixing mechanism.

3. The extraction apparatus according to claim 1, **characterized in that** the sample processing device further comprises a processing box comprising two sets of processing chambers samples.

4. The extraction apparatus according to claim 3, **characterized in that** the magnetic attraction and mixing mechanism comprises a mixing assembly and a magnetic assembly, the magnetic assembly comprising a magnet disposed on at least one side of the mixing assembly, and the processing box being disposed on the mixing assembly.

5. The extraction apparatus according to claim 4, **characterized in that** the magnet comprises a first magnet and a second magnet, the first magnet and the second magnet being respectively disposed on two sides of the mixing assembly and respectively located on sides of the two sets of processing chambers that are away from each other.

6. The extraction apparatus according to claim 4, **characterized in that** the magnetic assembly further comprises a magnet driving mechanism, the magnet driving mechanism comprising a first driving motor, a lead screw and a linkage, wherein the lead screw is drivingly connected to the first driving motor, one end of the linkage is connected to the lead screw and the other end of the linkage is connected to the magnet, and the magnet is driven by the first driving motor to extend above or retract below an upper side surface of the magnetic attraction and mixing mechanism.

7. The extraction apparatus according to claim 4, **characterized in that** the mixing assembly comprises a second driving motor and a mixing platform, the mixing platform comprising an eccentric shaft to which the second driving motor is drivingly connected.

8. The extraction apparatus according to claim 1, **characterized in that** the number of sample processing devices is one or plural.

9. The extraction apparatus according to claim 1, **characterized in that** the consumable assembly further comprises a reagent kit and a pipette tip box, the reagent kit comprising one or more cavities for containing reagents, and the pipette tip box comprising one or more pipette tips.

10. The extraction apparatus according to claim 1, **characterized in that** two first guide rails are provided, which are disposed opposite each other, and between which the second guide rail is disposed.

11. The extraction apparatus according to claim 10, **characterized in that** the mechanical arm device further comprises a first synchronizing belt, a first pulley, and a first guide rail motor, wherein the first synchronizing belt is disposed along the first guide rail, the first pulley is fixed at one end of the first synchronizing belt, the first guide rail motor drives the first pulley to rotate, the first pulley drives the first synchronizing belt to move, and the second guide rail is capable of being driven by the first synchronizing belt to move in a direction perpendicular to the first guide rail.

12. The extraction apparatus according to claim 1, **characterized in that** the mechanical arm device further comprises a second synchronizing belt, a second pulley and a second guide rail motor, wherein the second guide rail motor is disposed on the operating arm, the second synchronizing belt is disposed along the second guide rail, the second pulley is fixed to the operating arm and movable along the second synchronizing belt, the second guide rail motor drives the second pulley to rotate such that the second pulley is moved along the second synchronizing belt, and the operating arm moves with the second pulley along the second guide rail.

13. The extraction apparatus according to claim 1, **characterized in that** the mechanical arm device further comprises a lifting motor and a lifting lead screw, wherein the operating arm is connected to the lifting lead screw, and the lifting motor drives the lifting lead screw to move and drives the operating arm to perform a lifting or lowering movement.

14. The extraction apparatus according to claim 1, **characterized in that** the first guide rail and the second guide rail are each provided with a position sensor.

15. The extraction apparatus according to claim 1, **characterized by** further comprising a waste liquid box provided with a waste liquid discharge channel.

16. The extraction apparatus according to claim 1, **characterized in that** the consumable assembly further comprises a target biological substance storage box.

17. A method of use of an extraction apparatus, **characterized by** comprising:
controlling a mechanical arm device to add a sample into a first processing chamber of a processing box and to add a first reagent into the first processing chamber;
controlling a mixing assembly to perform a first mixing on the processing box;
controlling the mechanical arm device to add first magnetic beads into the first processing chamber;
controlling a magnetic assembly to perform a first magnetic attraction on the first magnetic beads in the first processing chamber;
controlling the mechanical arm device to transfer a first supernatant from the first processing chamber to a second processing chamber of the processing box and to add second magnetic beads into the second processing chamber;
controlling the mixing assembly to perform a second mixing on the processing box;
controlling the magnetic assembly to perform a second magnetic attraction on the second magnetic beads in the second processing chamber;
controlling the mechanical arm device to remove a second supernatant from the second processing chamber while retaining the attracted second magnetic beads; and
performing elution on the second magnetic beads and controlling the mechanical arm device to collect a third supernatant from the second processing chamber.

18. The method of use according to claim 17, **characterized in that** controlling a mixing assembly to perform a first mixing on the processing box comprises: controlling a second driving motor to rotate at a first preset rotation speed for a first preset period of time to mix the first reagent with the sample.

19. The method of use according to claim 18, **characterized in that** the first reagent may comprise a first sub-reagent and a second sub-reagent, the first preset rotation speed may comprise a first preset sub-rotation speed and a second preset sub-rotation speed, and the first preset period of time may comprise a first preset sub-period of time and a second preset sub-period of time; and controlling a second driving motor to rotate at a first preset rotation speed for a first preset period of time to mix the first reagent with the sample comprises:
adding the first sub-reagent, and controlling the second driving motor to rotate at the first preset sub-rotation speed for the first preset sub-period of time to mix the first sub-reagent with the sample; and
adding the second sub-reagent after the first preset period of time expires, and controlling the second driving motor to rotate at the second preset sub-rotation speed for the second preset sub-period of time to mix the second sub-reagent with the sample mixed with the first sub-reagent.

20. The method of use according to claim 17, **characterized in that** controlling the mechanical arm device to add first magnetic beads into the first processing chamber comprises:
controlling the second driving motor to rotate at a second preset rotation speed for a second preset period of time such that the first magnetic beads are conjugated to impurities.

21. The method of use according to claim 17, **characterized in that** controlling a magnetic assembly to perform a first magnetic attraction on the first magnetic beads in the first processing chamber comprises:
controlling a first driving motor to extend a first magnet above an upper side surface of a mixing platform, to use a magnetic force of the first magnet to attract the first magnetic beads to a side wall of the first processing chamber, and standing for a third preset period of time.

22. The method of use according to claim 17, **characterized in that** controlling the mixing assembly to perform a second mixing on the processing box comprises:
controlling a second driving motor to rotate at a third preset rotation speed for a fourth preset period of time to conjugate the second magnetic beads to a target biological substance.

23. The method of use according to claim 17, **characterized in that** controlling the magnetic assembly to perform a second magnetic attraction on the second magnetic beads in the second processing chamber comprises:
controlling a first driving motor to extend the second magnet above an upper side surface of a mixing platform, to use a magnetic force of the second magnet to attract the second magnetic beads conjugated to the target biological substance to a side wall of the second processing chamber, and standing for a fifth preset period of time.

24. The method of use according to claim 17, **characterized in that** performing elution on the second magnetic beads and controlling the mechanical arm device to collect a third supernatant from the second processing chamber comprises:
controlling the magnetic assembly to retract the second magnet below an upper side surface of a mixing platform, and controlling the mechanical arm device to add a second reagent into the second processing chamber, the second reagent being capable of eluting a target biological substance from the second magnetic beads;
controlling the mixing assembly to perform a third mixing on the processing box;
controlling the magnetic assembly to perform a third magnetic attraction on the second magnetic beads in the second processing chamber, and standing for a sixth preset period of time; and
controlling the mechanical arm device to collect the third supernatant from the second processing chamber.
